# EUROPÄISCHE PATENTANMELDUNG

(11) **EP 4 729 562 A1**
(43) Veröffentlichungstag der Anmeldung: **22.04.2026**
(21) Anmeldenummer: 24207770.9
(22) Anmeldetag: 21.10.2024
(51) Int. Cl.: C08G 71/04, A61B 6/10, C08L 75/06, G21F 1/00

(54) **BESCHICHTUNGSZUSAMMENSETZUNG**

(71) Anmelder: MAVIG GmbH, 81829 München (DE)
(72) Erfinder: STOIAN, Christian, 81829 München (DE)
(74) Vertreter: Schwarz, Claudia

(57) **Zusammenfassung**

**Beschichtungszusammensetzung,** umfassend eine wasserbasierte Polyester- aliphatische Polyurethan-Dispersion, enthaltend isocyanatfreie Polyurethane (NIPU), **dadurch gekennzeichnet,** dass der Feststoffgehalt (d.h. die aliphatischen Polyester-Polyurethane) der Dispersion im Bereich von 50 bis 95 Gew.-% liegt und dass diese durch Trocknung aushärtet.

## Beschreibung

Die Erfindung betrifft eine umweltfreundliche und isocyanatfreie Beschichtungszusammensetzung auf Basis von aliphatischen Polyester-Polyurethanen, die zur Beschichtung von Materialien für den Strahlenschutz verwendet wird. Die Erfindung betrifft ferner die Beschichtungszusammensetzung, die ferner spezielle chemische Elemente enthält, die biozide, insbesondere mikrobiozide Eigenschaften aufweisen und/oder in der Lage sind, ionisierende Strahlung abzuschirmen. Darüber hinaus betrifft die Erfindung auch Strahlenschutzmaterialien, das mit der der erfindungsgemäßen Zusammensetzung beschichtet oder hergestellt wird, sowie die Herstellung von Strahlenschutzmaterialien unter Verwendung der erfindungsgemäßen Beschichtungszusammensetzung.

Strahlenschutzmaterialien dienen dem Schutz von Personen und Geräten vor den schädlichen Auswirkungen ionisierender Strahlung. Sie gewährleisten die Sicherheit und Gesundheit von Personen, bei Anwendungen, in denen eine hohe Strahlungsabschirmung erforderlich ist, wie beispielsweise in der Medizintechnik, in der Nuklearindustrie oder in anderen Bereichen, in denen ionisierende Strahlung eine Rolle spielt.

Zum Beispiel sind in Krankenhäusern und Arztpraxen Strahlenschutzmaterialien in jeglicher Form im Einsatz, um Patienten und medizinisches Personal vor hochenergetischer Strahlung zu schützen, die bei diagnostischen Verfahren wie Röntgen, Computertomographie oder Strahlentherapie verwendet wird. Beispiele solcher aus Strahlenschutzmaterialien gebildeter Artikel sind vielfältig und beinhalten Bekleidung, Handschuhe, Schürzen, Gonadenschutz, sowie Wandbehänge, Vorhänge und Untertischschutz.

Strahlenschutzmaterial beinhaltet immer ein oder mehrere schwere chemische Elemente in einer ausreichenden Konzentration, die die energetische Strahlung aufnehmen und/oder bremsen. Sowie einer Matrix oder einem beliebigen, der Anwendung angepassten Trägermaterial. Die am häufigsten verwendete Matrix besteht aus Elastomere, z.B. vulkanisiertem Naturkautschuk (NR), Synthesekautschuk (BR) oder chlorsulfoniertem Polyethylen (CSM).

Als schwere chemische Elemente können grundsätzlich Folgende verwendet werden: Blei (Pb), Bismut (Bi), Wolfram (W), Zinn (Sn), Antimon (Sb) und Barium (Ba). Blei und Bismut sind die derzeit am häufigsten im Strahlenschutz verwendeten Elemente.

Von den vorgenannten Materialien ist Bleigummi das derzeit meistgenutzte Strahlenschutzmaterial. Er besteht aus vulkanisiertem Naturkautschuk oder Synthesekautschuk und enthält als schweres Element Blei in elementarer, ionischer, komplexierter Form oder als Oxid.

Die Menge an mindestens einem schweren chemischen Element im Strahlenschutzmaterial entscheidet darüber, wie hoch die Schutzwirkung ist. Diese Schutzwirkung wird als Bleigleichwert angegeben. Bleigleichwert ist eine Maßeinheit, die die Abschirmwirkung eines Materials gegen ionisierende Strahlung beschreibt. Er gibt an, wie viel Millimeter Blei (Pb) erforderlich wären, um die gleiche Abschirmwirkung zu erzielen wie das betreffende Material. Der Bleigleichwert wird in Millimetern Blei (mm Pb) angegeben und dient vor allem in Bereichen des Strahlenschutzes, wie bei Röntgenschürzen oder Schutzwänden, als Vergleichsgröße, um die Effektivität unterschiedlicher Materialien hinsichtlich der Strahlenabsorption zu bewerten. Je höher der Bleigleichwert, desto besser ist die Abschirmung gegen Strahlung.

Strahlenschutzmaterial muss neben dem erforderlichen Bleigleichwert auch eine hohe Beständigkeit gegen mechanische und chemische Beanspruchung und auch Alterung aufweisen. Diese Korrosionsbeständigkeit wird durch das Aufbringen einer schützenden Schicht auf den oder der Oberflächen des Strahlenschutzmaterials erreicht. Diese Schicht kann eine Folientasche sein oder Schutzschicht. Folientaschen werden z.B. in EP 3748 652 A1 und EP 1613 217 A1 beschrieben und sind derzeit die am Häufigsten verwendeten Mittel, um die Oberflächen von Bleigummi zu schützen.

Neben der Bildung von Löchern und Rissen an den Oberflächen sind die derzeit verwendeten Taschen besonders an den Nähten empfindlich und hohe mechanische Beanspruchung führt dazu, dass die Naht aufreißt. Die Beschichtungen haben das Problem, dass sie sich vom Substrat (dem Strahlenschutzmaterial) ablösen können. Vor allem kommt es an den Kanten des Materials zu vorzeitiger Korrosion, Abnutzung und Beschädigung. Das hängt unter anderem auch damit zusammen, dass an diesen Stellen häufig das Phänomen der sogenannten "Kantenflucht" auftritt. Das bedeutet, dass an den Kanten des Werkstücks die Beschichtung dünner aufgetragen wird oder abläuft, wodurch eine unzureichende Deckung entsteht. Die Kantenflucht wird vor allem durch physikalische Effekte wie Oberflächenspannung und Schichtdicke beeinflusst.

Die Herstellung und die Entsorgung von Strahlenschutzmaterial sind aus ökologischen Gründen nicht vorteilhaft. Bei der Beschichtung werden oft Systeme verwendet, die Lösungsmittel enthalten und Isocyanate verwenden, wodurch bei der Herstellung besondere Schutzmaßnahmen für Mensch und Umwelt getroffen werden müssen. Gleichfalls gibt es noch keine wirtschaftlich relevante Möglichkeit, beschädigte Strahlenschutzprodukte zu recyclen und die Entsorgung ist kompliziert. Sie müssen nämlich aufgrund der schweren chemischen Elementen (z.B. Blei) als Sondermüll vernichtet werden. Bislang sind noch keine Möglichkeiten zum Recyclieren gefunden.

Ziel der vorliegenden Erfindung ist es Beschichtungszusammensetzungen, sowie Methoden zur Beschichtung und Herstellung von Strahlenschutzmaterialien zu finden, mit denen die Nachteile des Standes der Technik vermieden werden können.

Die Erfinder haben es sich insbesondere zur Aufgabe gemacht, umweltfreundliche, Beschichtungszusammensetzungen und Verfahren für die Beschichtung und Herstellung von Strahlenschutzmaterial bereitzustellen, die widerstandsfähig (beständig) sind und die zumindest teilweise recyclebar sind.

Die Erfinder haben festgestellt, dass zur Lösung der gestellten Aufgabe Beschichtungszusammensetzungen besonders geeignet sind, die im Wesentlichen physikalisch, insbesondere durch Trocknung, aushärten. Solche Beschichtungszusammensetzungen enthalten Plastomere als Beschichtungsstoffe. Ausgehärtete Plastomere sind in geeigneten Lösemitteln löslich und damit recyclingfähig.

Die Erfinder haben eine Beschichtungszusammensetzung auf Wasserbasis gefunden, die sich hervorragend zum Beschichten von Substraten insbesondere elastischen und/oder starren Materialien (z.B. Gummi, Ton, Ziegel, Steine, Kunststeine, Zement etc.), und anderen Trägermaterialien (z.B. Textilien, Vliese, Mikrofasergewebe, usw.) eignet.

Durch Zusatz geeigneter Chemikalien und /oder chemischer Elemente kann die Beschichtungszusammensetzung auch eine biozide Wirkung entfalten. Solche Elemente sind z.B. Silber und Kupfer in jeglicher Form (elementar, ionisch, komplexiert, oxidiert).

Enthält die Beschichtungszusammensetzung und/oder das Substrat mindestens eines der strahlenabschirmenden schweren chemischen Elemente in beliebiger Form (elementar, ionisch, komplexiert, oxidiert), insbesondere Blei (Pb), Bismut (Bi), Wolfram (W), Zinn (Sn), Antimon (Sb) und Barium (Ba), so entsteht durch die Beschichtung erfindungsgemäßes Strahlenschutzmaterial.

Gleichfalls haben die Erfinder gefunden, dass die Beschichtungszusammensetzung mit vorteilhaften optischen und/oder benutzerfreundlichen Eigenschaften (wie z.B. einer angenehmen Haptik und/oder einer ansprechenden Optik durch Farbgestaltung) ausgestattet werden kann und diese aufweist, was für die Marktakzeptanz des mit dem beschichteten Strahlenschutzmaterial hergestellten Produkts vorteilhaft ist.

Die vorliegende Erfindung betrifft daher eine Beschichtungszusammensetzung, enthaltend eine aliphatische Polyester-Polyurethan-Dispersion auf Wasserbasis, wobei sie frei von Isocyanaten ist und nach dem Aushärten durch Trocknen eine Shore A Härte im Bereich von 50 bis 70 aufweist.

Die vorliegende Erfindung betrifft ferner die Verwendung der erfindungsgemäßen Beschichtungszusammensetzung zur Beschichtung von Strahlenschutzsubstraten (insbesondere von mit Blei oder Bismut versetzten Elastomeren), wobei die Beschichtungszusammensetzung einen Feststoffgehalt im Bereich von 50 bis 95 Gew.-%, bevorzugt im Bereich von 50 bis 70 Gew.-% und eine kinematische Viskosität bei 25 °C nach DIN 53211 im Bereich von 10 bis 40 Sekunden, vorteilhafterweise im Bereich von 15 bis 22 Sekunden aufweist.

Der Feststoffgehalt in Gew.-% bezieht sich auf den Gehalt an aliphatischen Polyester-Polyurethanen und nicht auf andere Bestandteile/Komponenten, die in der Beschichtungszusammensetzung enthalten sein können.

Die vorliegende Erfindung betrifft auch die Verwendung der erfindungsgemäßen Beschichtungszusammensetzung zur Herstellung von Strahlenschutzmaterialien durch Gießen und Aushärten der Beschichtungszusammensetzung, enthaltend mindestens ein schweres chemisches Element und ggf. Fasern (z. B. aus Glasfasern, Zementfasern, Fasern aus Ca₂CO₃, Textilfasern, Carbonfasern, Kunststofffasern usw.), wobei die Beschichtungszusammensetzung dann einen Feststoffgehalt im Bereich von 50 bis 95 Gew.-% , bevorzugt 50 bis 70 Gew.-% und eine kinematische Viskosität bei 25 °C nach DIN 53211 im Bereich von 10 bis 40 Sekunden aufweist.

Die aliphatischen Polyester-Polyurethane sind so ausgewählt, dass die resultierende Schicht auf dem Strahlenschutzmaterial eine Shore A-Härte im Bereich von 50 bis 70 aufweist. Dies bedeutet, dass die resultierende Schicht eine gute Härte und Beständigkeit gegen mechanische Beanspruchung aufweist, was sie für eine Vielzahl von Anwendungen geeignet macht.

Wird die Beschichtungszusammensetzung zur Herstellung von Strahlenschutzmaterial verwendet, so kann das mit der Beschichtungszusammensetzung hergestellte Strahlenschutzmaterial als Substrat für weitere Beschichtungen dienen. Diese Beschichtungen können funktionelle, haptische oder optische Eigenschaften aufweisen. Funktionelle Eigenschaften beziehen sich auf Oberflächeneigenschaften, wie beispielsweise biologische (biozide), physikalische (energiereiche Strahlen absorbierende), chemische (inerte) und mechanische Eigenschaften (z.B. die Härte der Oberfläche).

Die erfindungsgemäße Beschichtungszusammensetzung kann auch weitere Komponenten enthalten. Dies sind z.B. biologisch wirksame organische oder anorganische Substanzen (z.B. solche mit biozider, insbesondere mikrobiozider, Wirkung, einschließlich biozid wirkender Metalle wie Silber und Kupfer (in elementarer, ionischer, komplexierter oder oxidischer Form), Verdickungsmittel zur Einstellung der Viskosität, thixotrope Zustände vermittelnde Substanzen wie z.B. Aerosil, Xanthan und Netzmittel zur Modulation der Oberflächenspannung. Diese Netzmittel sind in der Regel amphotere Moleküle wie z.B. Tenside oder Alkohole, insbesondere Methanol, Ethanol, n-Propanol, iso-Propanol, Butanole.

Biozid bezeichnet die Eigenschaft eines Stoffes, schädliche Organismen wie Bakterien, Viren oder Pilze sowie Insekten zu bekämpfen (zu dezimieren oder abzutöten). Mikrobiozid bezeichnet die Eigenschaft, Mikroorganismen wie Bakterien oder Pilze gezielt abzutöten oder in ihrem Wachstum zu hemmen. Eine biozide, insbesondere mikrobiozide Beschichtung ist insbesondere dann von Vorteil, wenn die mit der Beschichtungszusammensetzung beschichteten oder konservierten Strahlenschutzmaterialien im Gesundheitswesen eingesetzt werden.

Die Beschichtungszusammensetzung kann ferner weitere Komponenten enthalten, die einen dekorativen oder funktionellen Zweck erfüllen. Dies können Farbmittel (z.B. synthetische anorganische Farbpigmente oder anisometrische Perlglanzpigmente, Xirallic^{™}-Pigmente, Metallic-Pigmente) oder Pigmente mit besonderen Eigenschaften (z.B. fluoreszierende, reflektierende, magnetische Partikel oder Nanopartikel) sein.

Die Beschichtungszusammensetzung ist nicht nur frei von Isocyanaten, sondern auch von anderen bedenklichen Stoffen wie Bisphenol A, das häufig als Weichmacher eingesetzt wird.

Bekannte und kommerziell erhältliche wässrige aliphatische Polyester-Polyurethan-Dispersionen, die keine Polyisocyanate enthalten, sind unter dem Handelsnamen RGO MediScin der Firma Lackiererei Lehner, Fürstenzell, Deutschland, erhältlich.

Wenn die ausgehärtete Beschichtungszusammensetzung ohne Verwendung von Substraten das Strahlenschutzmaterial bildet, können zusätzlich Fasern (z. B. aus Glasfasern, Zementfasern, Fasern aus Ca₂CO₃, Textilfasern, Carbonfasern, Kunststofffasern usw.) vorhanden sein.

Darüber hinaus kann die erfindungsgemäße Beschichtungszusammensetzung mindestens ein schweres chemisches Element enthalten, das zum Abschirmen von ionisierender Strahlung verwendet wird. Beispiele dafür solche schweren Elemente sind Blei (Pb), Bismut (Bi), Wolfram (W), Zinn (Sn), Antimon (Sb) und Barium (Ba). Diese Elemente können elementar, in komplexierter Form oder in ionischer Form wie z.B. Salze oder als Oxide eingesetzt werden. Erfindungsgemäß bevorzugt sind Blei und Bismut und deren Gemische verwendet. Durch den Zusatz von mindestens einem schweren chemischen Element in der Beschichtungszusammensetzung wird die Abschirmeigenschaft gegen ionisierende Strahlung erreicht.

Wenn nicht anders angegeben, bezieht sich der Begriff "Beschichtungszusammensetzung" auch auf solche Zusammensetzungen, die eine Strahlenschutzwirkung haben.

Die in der Beschichtungszusammensetzung enthaltenen schweren chemischen Elemente können je nach Verwendungszweck ausgewählt werden. Aus gesundheitlichen Gründen ist Bismut als schweres chemisches Element vorzuziehen. Und für den Fall, dass eine biozide Eigenschaft erwünscht ist, ist Kupfer im Vergleich zu Silber zu bevorzugen.

Die Beschichtungszusammensetzung zur Beschichtung von Strahlenschutzmaterial weist vorzugsweise eine kinematische Viskosität auf (gemessen in Centistokes (cSt) gemäß DIN 53211 mit einem DIN-4-Becher) und liegt im Bereich von 10 bis 40 Sekunden, vorteilhafterweise im Bereich von 15 bis 22 Sekunden bei 25°C.

Enthält die Beschichtungszusammensetzung schwere chemische Elemente, die in der Lage sind, Strahlung abzuschirmen, so ist die mit dieser Zusammensetzung erhaltene Schicht (im Folgenden als Strahlenschutzbeschichtung bezeichnet) in der Lage, ionisierende Strahlung abzuschirmen.

Diese Strahlenschutzbeschichtung kann ein- oder mehrschichtig aufgebaut sein. Die Konzentration des mindestens einen schweren chemischen Elements in der Beschichtungszusammensetzung sowie die Anzahl der aufgetragenen Einzelschichten bestimmen den Bleigleichwert der Strahlenschutzbeschichtung.

Die Strahlenschutzbeschichtung kann auf ein Strahlenschutzmaterial aufgebracht werden, was den Vorteil hat, dass dadurch der Bleigleichwert des verwendeten Strahlenschutzmaterials moduliert werden kann. Die Modulation wird durch die Konzentration des mindestens einen schweren chemischen Elements und die Anzahl der Schichten erreicht.

Die Kombination von Strahlenschutzmaterial und Strahlenschutzbeschichtung hat den Vorteil, dass verschiedene schwere chemische Elemente kombiniert werden können. Beispielsweise kann das Strahlenschutzmaterial Blei und die Strahlenschutzbeschichtung Bismut enthalten.

Eine solche Kombination hat den Vorteil, dass bei Verwendung von weniger Blei im Strahlenschutzmaterial das beschichtete Endprodukt bei ausreichender Bleigleichwertigkeit ein geringeres Gewicht aufweist. Ein geringes Gewicht des Strahlenschutzmaterials ist z. B. bei Produkten relevant, die von Personen am Körper getragen werden (z. B. Schürzen).

Eine solche Kombination hat weiterhin den Vorteil, dass das in der Beschichtung verwendete schwere Element beim Recycling der Beschichtung abgeschieden und wiederverwendet werden kann.

Diese Strahlenschutzbeschichtung kann auf beliebige Substrate insbesondere elastische oder starre Materialien (z.B. Gummi, Ton, Ziegel, Steine, Kunststeine, Zement etc.), und andere Trägermaterialien (z.B. Textilien, Vliese, Mikrofasergewebe, etc.) aufgebracht werden. Auf diese Weise können vielseitig einsetzbare neuartige Strahlenschutzvorrichtungen und Strahlenschutzmaterialien hergestellt werden.

Die mindestens ein schweres chemisches Element enthaltende Beschichtungszusammensetzung kann ohne Verwendung eines Substrats und eines Trägermaterials zu einem Strahlenschutzmaterial verarbeitet werden, dessen Oberflächen wie hier beschrieben weiter behandelt werden können. Die Herstellung des substratlosen Strahlenschutzmaterials erfolgt vorteilhafterweise nach einem Kaltvergussverfahren (Kaltgießverfahren).

Die Beschichtung entsteht durch physikalische Aushärtung der Beschichtungszusammensetzung, d.h. es sind keine chemischen Reaktionen zur Schichtbildung erforderlich.

Die Beschichtung von Substraten oder Trägermaterialien kann allseitig oder einseitig erfolgen. Sofern nicht anders angegeben, erfolgt die Beschichtung allseitig. Die Beschichtung weist eine besonders gute Haftung und Haftfestigkeit auf dem Substrat oder Trägermaterial auf. Die Haftfestigkeit ist ein Maß für den Widerstand einer Beschichtung gegen ihre mechanische Ablösung vom Substrat ist. Die Beschichtung weist auch eine hervorragende Verformbarkeit (Flexibilität) auf.

Die Erfinder haben zudem erkannt, dass eine Vorbehandlung der Substrate oder Trägermaterialien vorteilhaft sein kann, um deren Kontaktaffinität zu erhöhen. Der Fachmann kann je nach verwendetem Substrat das geeignete Verfahren auswählen. Wenn Textilien als Substrat verwendet werden, kann es vorteilhaft sein, die Textilien mit Haftvermittlern zu behandeln.

Ebenso kann eine Reinigung der Oberfläche des Substrats oder Trägermaterials vor dem Beschichten von Vorteil sein.

Handelt es sich bei dem zu beschichtenden Substrat um den sogenannten Bleigummi, d.h. Blei in einer elastischen Matrix, dann ist es die Beständigkeit der Beschichtung von Vorteil, wenn die zu beschichtende Oberfläche frei von Verunreinigungen (z.B. Talkum) ist. Zur Entfernung des Talkums wird das Substrat vorzugsweise nass gereinigt.

Diese Reinigungsflüssigkeit basiert - auch wegen der guten Umweltverträglichkeit - auf Wasser. Diesem können weitere waschaktive Substanzen zugesetzt werden. Solche waschaktiven Substanzen sind alkalische Lösungen wie Natronlauge oder Seifen und geeignete Reinigungsmittel.

Bei Bleigummi, der beschichtet werden soll, hat es sich ebenfalls als vorteilhaft für die Haftung der Beschichtung auf dem Substrat erwiesen, wenn die Oberfläche des Bleigummis vor, nach oder während des Waschvorgangs mechanisch gereinigt wird. Zum Beispiel mit einer Bürste oder ähnlichem.

Um die Entstehung von Abriebstaub bei der mechanischen Reinigung des Substrates zu vermeiden, ist es von Vorteil, wenn diese während des Waschens erfolgt. Ein weiterer Vorteil besteht darin, dass sich der Abrieb dann in der Waschlösung befindet und dieser Abrieb sehr gut durch Filtration oder Sedimentation von der Waschlösung getrennt und separat entsorgt oder recycelt werden kann.

Neben den gereinigten Oberflächen des Substrats oder des Trägermaterials kann es für die Haftfestigkeit der erfindungsgemäßen Beschichtung von Vorteil sein, wenn die Oberflächenspannung des Substrats oder des Trägermaterials durch geeignete Maßnahmen moduliert, vorzugsweise erhöht wird.

Bei nicht textilen Substratoberflächen können dies Plasma- oder Laserbehandlungen, chemische Oberflächenbehandlungen einschließlich Ätzen und Ultraschallbehandlungen sein.

Für die Oberflächen von textilen Substraten eignen sich vor allem chemische Oberflächenbehandlungen und gegebenenfalls Ätzungen oder Ultraschallbehandlungen. Der Fachmann kann leicht feststellen, welche Oberflächenbehandlung für das jeweils verwendete Substrat vorteilhaft ist.

Handelt es sich bei dem Substrat um Bleigummi, so hat es sich als vorteilhaft erwiesen, nach der Reinigung der Oberfläche (durch wässrige und/oder mechanische Reinigung) eine Plasmabehandlung durchzuführen. Dies kann vor oder nach dem Trocknen der Substratoberfläche erfolgen.

Zur Verbesserung der Haftfestigkeit können auf die Oberfläche des Substrats oder des Trägermaterials Haftvermittler aufgebracht werden. Es hat sich insbesondere bei Bleigummi als Substrat als vorteilhaft erwiesen, vor der Beschichtung einen Haftvermittler auf das Substrat aufzubringen. Der Haftvermittler kann aufgesprüht werden. Für Bleigummi geeignete Haftvermittler sind z.B. Kunststoffhaftvermittler wie RGO 1K Kunststoffhaftvermittler der Firma Lackiererei Lehner, Fürstenzell, Deutschland, ein Gemisch aus Ethylacetat, Xylol und leicht aromatischem Lösungsmittel Naphtha (Erdöl) (CAS: 64742-95-6). Der Fachmann kann leicht feststellen, welche Haftvermittler für das jeweilige Substrat oder Trägermaterials geeignet sind.

Vor der Beschichtung mit der erfindungsgemäßen Zusammensetzung darf die Oberfläche des Substrats oder Trägermaterial nur eine geringe Feuchtigkeit aufweisen. Diese geringe Feuchtigkeit (Rest- oder Objektfeuchte) ist kleiner oder gleich 5 %, bevorzugt kleiner oder gleich 4 %. Diese Objektfeuchte wird mit üblichen Geräten gemessen, wobei die Messung in der Regel auf Spannungsmessung oder magnetischer Messung beruht. Solche Geräte werden bspw. von den Firmen Voltkraft und TROTEC vertrieben (z.B. Gerät TROTEC BM12, HCHO/TVOC).

Die Beschichtung des Substrats bzw. Trägermaterials erfolgt durch einseitiges oder allseitiges, gegebenenfalls mehrfaches Auftragen der Beschichtungszusammensetzung. Die Applikation erfolgt durch den Fachmann bekannte Verfahren, wie z.B. Spritztechnik, Rakelzug, Niederdruckverfahren, Schleuderradverfahren, Walzverfahren, Kastenzug, Hochrotationsspritzen, Druckverfahren, Pumpsprühverfahren, Tauchverfahren, Airlessverfahren, Druckkesselverfahren HVLP modifiziert.

Diese Beschichtungen können bereits nach einmaligem Auftragen der Beschichtungszusammensetzung verwendet werden. Je nach Substrat bzw. Trägermaterial und Verwendung des beschichteten Strahlenschutzmaterials kann es vorteilhaft sein, die Beschichtungszusammensetzung mindestens zweimal, vorteilhafterweise mindestens vier oder fünfmal aufzutragen. Die Schichten können ohne Zwischentrocknung aufgetragen werden. Die Schichtdicke einer mit der erfindungsgemäßen Zusammensetzung erhaltenen Schicht liegt im Bereich von 0,1 bis 5 mm, oder im Bereich von 0,1 mm bis 2,0 mm, oder im Bereich von 0,4 mm bis 2 mm, oder im Bereich von 0,6 bis 3,0 oder 5,00 mm, oder im Bereich von 0,8 bis 4,0 mm.

Weist das Substrat Aussparungen auf, z.B. vorgestanzte Löcher für die spätere Befestigung an einer Vorrichtung, so werden diese mitbeschichtet und auch hier ist die Kantenflucht vernachlässigbar.

Die Beschichtungszusammensetzung wird bei Raumtemperatur, insbesondere bei einer Temperatur im Bereich von 18 bis 25 °C aufgetragen oder, wenn kein Substrat oder Trägermaterial verwendet wird, gegossen. Der Temperaturbereich ist so gewählt, dass die Verarbeitung bei nordeuropäischer Umgebungstemperatur, d.h. ohne Heizen oder Kühlen der Umgebung stattfinden kann, was das Verfahren energiesparend macht.

Die Trocknung der Substrate nach der wässrigen Reinigung und vor der Beschichtung sowie die Verfestigung der Beschichtungszusammensetzung erfolgt durch physikalische Trocknung.

Abhängig von der vorherrschenden Luftfeuchtigkeit beträgt die Trocknungszeit bei Raumtemperatur bis zu 20 Stunden. Die Verfestigung, d.h. die physikalische Trocknung der Beschichtungszusammensetzung ist der Übergang vom fließfähigen/flüssigen in den festen Zustand unter Abgabe der Solventen, hier vorüberwiegend Wasser.

Die Erfinder haben festgestellt, dass die Trocknung und die Verfestigung durch verschiedene Maßnahmen beschleunigt werden kann, ohne die Qualität der resultierenden Beschichtung negativ zu beeinflussen.

Bei diesen Maßnahmen handelt es sich im Wesentlichen um eine Kälte- oder Wärmeeinwirkung sowie um eine kombinierte Wärme- und Kälteeinwirkung (Kombinationswärme). Durch kombinierte Wärme- und Kälteeinwirkung kann die Trocknungszeit - je nach vorherrschender Luftfeuchtigkeit in der Kammer - auf wenige Stunden, d. h. auf weniger als 4 Stunden, oft sogar auf weniger als 2 Stunden verkürzt werden. Die Temperatur für die Wärmeeinwirkung wird an das Substrat- und Beschichtungsmaterial angepasst und kann bis zu 80 °C betragen.

Es hat sich auch als vorteilhaft erwiesen, wenn die Kanten des beschichteten Substrats bei der Trocknung / Verfestigung freistehen, um einen verbesserten Beschichtungsumgriff zu erhalten.

Die Oberflächen der getrockneten Beschichtungszusammensetzung können - je nach Wunsch des Verwenders - auch individualisiert werden. So können sie z.B. eingefärbt oder mit Latex oder UV-Printfarben bedruckt werden.

Auf die Oberflächen der getrockneten Beschichtungszusammensetzung können auch nachträglich Befestigungs- oder Verschlussvorrichtungen oder andere für die Verwendung nützliche Komponenten aufgebracht werden. Solche Komponenten sind zum Beispiel Haltefahnen, Befestigungslaschen, Knöpfe oder Reißverschlüsse usw. Die Befestigung kann durch thermisches Aufschweißen erfolgen.

Das mit der Beschichtungszusammensetzung hergestellte oder beschichtete Strahlenschutzmaterial weist eine ausgezeichnete Verformbarkeit auf. Die Beschichtung weist eine ausgezeichnete Haftfestigkeit auf dem Substrat auf, d.h. sie besitzt einen hohen Widerstand gegen mechanische Ablösung vom Substrat.

Das mit der Beschichtungszusammensetzung hergestellte oder beschichtete Strahlenschutzmaterial kann unterschiedlich eingesetzt werden. So eignet sich beschichteter Bleigummi z. B. zur Herstellung von Lamellen für einen Untertisch-Strahlenschutz oder für einen Vorhang oder für eine Kassettenabdeckung oder einen Abdeckwinkel. Mit der Strahlenschutzbeschichtung beschichtete Trägermaterialien (z. B. Vliese oder Mikrofasergewebe, Kunstleder) eignen sich zur Herstellung von Strahlenschutzkleidung, z. B. Schürzen, Handschuhe, Kittel, Gonadenschutz, Überschuhe etc.

Die Erfindung wird durch das nachfolgende Beispiel erläutert, ohne jedoch auf dieses Beispiel beschränkt zu sein.

### Exemplarische Herstellung einer beschichteten Bleigummiplatte

Als Trägermaterial werden Bleigummiplatten mit einer Dicke von 0,5 mm verwendet. Diese werden vor der Beschichtung mit einer alkalischen Waschlösung von Talkum und anderen Verunreinigungen befreit. Nach dem Trocknen werden sie einer Plasmabehandlung durch Beflammen unterzogen.

Anschließend werden sie mit dem Kunststoffhaftvermittler RGO 1K der Firma Lackiererei Lehner, einem schnelltrocknenden Haftvermittler auf Ethylacetatbasis, behandelt und mit der Beschichtungszusammensetzung beschichtet.

Die Beschichtungszusammensetzung umfasst eine viskose, wässrige aliphatische Polyester-Polyurethan-Dispersion mit einem Feststoffgehalt von ca. 60 Gew.-% und einem pH-Wert im Bereich von 7 bis 8. Die Dichte der Zusammensetzung beträgt 1,05 g/cm3.

Die Beschichtung wird mehrmals (bis zu 15-mal) wiederholt, danach erfolgt die physikalische Trocknung. Nach Beendigung der Beschichtung ist die Beschichtung nach 10 - 20 Minuten, gemessen bei einer relativen Luftfeuchtigkeit von 40 - 65 %, ausgehärtet und nach 18 Stunden durchgetrocknet.

Die so erhaltene Beschichtung hat eine nach DIN 53505 gemessene Shore A-Härte von 60 und eine König-Härte von 20s.

Die kinematische Viskosität der Beschichtungszusammensetzung bei 15-22 sec. CP/25°C (Centi Stockes) DIN53211 DIN4

### Exemplarisches Prüfen der Stabilität der Beschichtung

Die Widerstandsfähigkeit gegen mechanische Beanspruchung und die Haftbeständigkeit der Schicht wurde wie nachfolgend beschrieben geprüft:

Es wurde ein Versuchsaufbau gewählt, bei dem eine wiederholte mechanische Beanspruchung eines erfindungsgemäß beschichteten Strahlenschutzmaterials durch Reibung und Schlag und Verdrehen an einem festen Hindernis durchgeführt wird.

Es wurde eine Strahlenschutzlamelle der Größe 30x20 cm verwendet, die als Substrat Bleigummi enthält und gemäß Beispiel 1 beschichtet ist (im Folgenden als Probe bezeichnet).

Als automatisiertes Bewegungssystem wurde ein industrieller FDM/FFF (Fused Deposition Modeling / Fused Filament Fabrication)-3D-Drucker Creality 3D-CR-10 Max verwendet. An der Vorrichtung zur Aufnahme des Extrusionskopfes des 3D-Druckers wurde eine Klemmhalterung angebracht, um damit die Probe stabil zu befestigen. Diese Aufnahmevorrichtung wird von einer Software gesteuert und von einem Antrieb bewegt und kann wiederholte Bewegungen ausführen. Sie wird durch eine programmierte Sequenz gesteuert, um die befestigte Probe und ihre Oberflächen und Kanten über oder gegen ein Hindernis zu schleifen oder zu stoßen und zu verdrehen.

Das bewegliche Hindernis kann auch gesteuert werden, was zu einer noch stärkeren Beanspruchung der Probe und damit zu einer höheren Intensität des Versuchs führt. Im vorliegenden Versuchsaufbau besteht das Hindernis aus einer 10-Liter-Weithals-Vierkantflasche aus HDPE mit Schraubverschluss. Sie wird stabil liegend auf einer vorzugsweise automatisch verfahrbaren Unterlage unter der Aufnahmevorrichtung des Extrusionskopfes montiert. Anstelle der gesteuerten Aufnahmevorrichtung des 3D-Druckers kann auch ein Roboterarm verwendet werden, an dem die Probe befestigt ist und der einem bestimmten programmierten Bewegungsmuster folgt. Das Bewegungsmuster des Kopfes und der Auflage, das im vorliegenden Versuch verwendet wird, ist so gewählt, dass die Stirnflächen der Lamelle und die Kanten entlang des Hindernisses schleifen (Reibungsversuch) und/oder in einem Winkel gegen das Hindernis stoßen (Aufprallversuch).

Die Bewegung ist auf 100 000 Zyklen programmiert. Jeder Zyklus besteht aus einer Bewegung, bei der die Probe mit dem Hindernis in Berührung kommt (Schleifen und Stoßen).

Nach verschiedenen Zeitabständen wurde die Oberfläche der Probe visuell kontrolliert.

Nach Abschluss des Tests wird die Oberfläche der Probe visuell auf Verschleiß, Risse, Ablösung der Beschichtung oder Verformung und Mikroskopische Aufnahmen getätigt, um Mikro-Risse oder kleinflächige Beschädigungen festzustellen, die mit bloßem Auge nicht sichtbar sind.

Nach der letzten Belastungsphase kann ein Penetrationstest durchgeführt werden, um zu überprüfen, ob die Beschichtung durch die mechanische Beanspruchung durchbrochen oder durchrieben wurde und ob die Bleiplatten freigelegt sind.

Zusammenfassend kann festgestellt werden, dass auch nach mehrmaliger Wiederholung des Versuchs und mit unterschiedlichen Proben keine nennenswerten Verformungen, Risse oder Ablösungen der beschichteten Oberfläche festgestellt werden konnten.

## Patentansprüche

1. **Beschichtungszusammensetzung,** enthaltend eine
wasserbasierte isocyanatfreie aliphatische Polyester-Polyurethan-Dispersion, **dadurch gekennzeichnet, dass**
- der Feststoffgehalt der aliphatischen Polyester-Polyurethane in der Dispersion im Bereich von 50 bis 95 Gew.-% liegt und
- dass die Dispersion durch Trocknung zu einer Beschichtung mit einer Shore A-Härte im Bereich von 50 bis 70 aushärtet.

2. **Beschichtungszusammensetzung** nach Anspruch 1, wobei deren kinematische Viskosität bei 25°C im Bereich von 10 bis 40 Sekunden liegt.

3. **Beschichtungszusammensetzung** nach Anspruch 1 oder 2, zusätzlich enthaltend Verdickungsmittel zur Einstellung der Viskosität, thixotrope Zustände vermittelnde Substanzen und/oder Netzmittel zur Modulation der Oberflächenspannung.

4. **Beschichtungszusammensetzung** nach einem der vorhergehenden Ansprüche, zusätzlich enthaltend biologisch wirksame organische oder anorganische Substanzen, insbesondere biozid wirksames Silber und/oder Kupfer, jeweils in elementarer Form, ionischer Form, in komplexierter Form oder in Form ihrer Oxide.

5. **Beschichtungszusammensetzung** nach einem der vorhergehenden Ansprüche, ferner umfassend mindestens ein schweres chemisches Element als Oxid oder in elementarer, ionischer oder komplexierter Form, das zum Abschirmen von ionisierender Strahlung verwendet wird ausgewählt unter Blei (Pb), Bismut (Bi), Wolfram (W), Zinn (Sn), Antimon (Sb) und Barium (Ba).

6. **Verwendung** einer Beschichtungszusammensetzung nach einem der vorhergehenden Ansprüche zum Beschichten eines elastischen Substrats, welches aus einem oder mehreren Materialien gebildet ist, ausgewählt unter vulkanisiertem Naturkautschuk (NR), Synthesekautschuk (BR) und chlorsulfoniertem Polyethylen (CSM), und wobei das Substrat ein oder mehrere schwere chemische Elemente enthält, die zum Abschirmen von ionisierender Strahlung verwendet werden, wobei diese chemischen Elemente ausgewählt sind unter Blei (Pb), Bismut (Bi), Wolfram (W), Zinn (Sn), Antimon (Sb) und Barium (Ba).

7. **Verwendung** einer Beschichtungszusammensetzung nach einem der Ansprüche 1 bis 5 zur Beschichtung von starren und/oder textilen Substraten, ausgewählt aus Ton, Ziegeln, Steinen, Kunststeinen, Zement, Textilien, Vliesen, Mikrofasergeweben, wobei entweder die Substrate oder die Beschichtungszusammensetzung ein oder mehrere schwere chemische Elemente umfasst, die zum Abschirmen von ionisierender Strahlung verwendet werden, wobei das schwere chemische Element oder die schweren chemischen Elemente ausgewählt sind aus Blei (Pb), Bismut (Bi), Wolfram (W), Zinn (Sn), Antimon (Sb) und Barium (Ba), die in elementarer, ionischer, oxidierter oder komplexierter Form verwendet werden.

8. **Verwendung** einer Beschichtungszusammensetzung, wie in Anspruch 5 definiert, zur Herstellung eines gegossenen Strahlenschutzmaterials, wobei die Beschichtungszusammensetzung ferner Fasern enthält, die ausgewählt sind unter aus Glasfasern, Zementfasern, Fasern aus Ca₂CO₃, Textilfasern, Carbonfasern, und Kunststofffasern.

9. **Verfahren** zur Herstellung eines beschichteten Strahlenschutzmaterials, wobei das Strahlenschutzmaterial aus einem elastischen Substrat besteht, welches aus einem oder mehreren Materialien gebildet wird, ausgewählt aus vulkanisiertem Naturkautschuk (NR), Synthesekautschuk (BR) und chlorsulfoniertem Polyethylen (CSM), wobei das Strahlenschutzmaterial ein oder mehrere schwere chemische Elemente enthält, die zum Abschirmen von ionisierender Strahlung verwendet werden, wobei das schwere chenmische Element oder die schweren chemischen Elemente ausgewählt sind unter Blei (Pb), Bismut (Bi), Wolfram (W), Zinn (Sn), Antimon (Sb) und Barium (Ba),
das die die folgenden Schritte umfasst:
(a) Reinigen der zu beschichtenden Oberflächen des Strahlenschutzmaterials mit einer wässrigen Lösung;
(b) Behandeln der zu beschichtenden Oberflächen des Strahlenschutzmaterials mit Plasmaflamme und/oder einem Haftvermittler;
(c) Trocknen der zu beschichtenden Oberflächen des Strahlenschutzmaterials auf eine Restfeuchte von kleiner oder gleich 5 %;
(d) ein oder mehrmaliges Auftragen der Beschichtungszusammensetzung, wie in einem der Ansprüche definiert, die auf die Beschichtungszusammensetzung gerichtet sind, auf die zu beschichtende Oberflächen des Strahlenschutzmaterials, wobei die Beschichtungsdicke der einen oder mehreren Schichten im Bereich von 0,1 bis 5 mm liegt;
(e) Aushärten der Beschichtung bei Temperaturen bis 85 °C.

10. Verfahren nach Anspruch 9, wobei die Trocknung durch eine kombinierte Einwirkung von Wärme und Kälte erfolgt.

11. Verfahren nach Anspruch 10, wobei auf die Beschichtung Befestigungsvorrichtungen oder Verschlussvorrichtung durch thermisches Aufschweißen aufgebracht sind.

12. Strahlenschutzmaterial, hergestellt nach dem Verfahren gemäß einem der Ansprüche 9 bis 11, wobei die Beschichtung eine Shore A-Härte im Bereich von 50 bis 70 aufweist.
